# EUROPÄISCHE PATENTSCHRIFT

(11) **EP 0 546 221 B1**
(45) Veröffentlichungstag und Bekanntmachung des Hinweises auf die Patenterteilung: **15.02.1995**
(21) Anmeldenummer: 91203259.6
(22) Anmeldetag: 11.12.1991
(51) Int. Cl.: A61M 29/02

(54) **Ballonkatheter**
Balloon catheter
Cathéter à ballonnet

(43) Veröffentlichungstag der Anmeldung: 16.06.1993
(73) Patentinhaber: SCHNEIDER (EUROPE) AG, 8180 Bülach (CH)
(72) Erfinder: Pfenninger, Susanne, CH-8610 Uster (CH)
(74) Vertreter: Misrachi, Alfred

(56) Entgegenhaltungen:
- EP-A- 0 441 384
- DE-U- 9 106 499

## Beschreibung

Die Erfindung betrifft einen Ballonkatheter mit einem Schaft, an dessen distalem Ende ein Ballon angebracht ist, wobei der Schaft ein Lumen aufweist, durch das der Ballon gespeist wird und ein Lumen durch das ein Führungsdraht geführt wird, mit dem der Katheter geführt und gesteuert wird, dabei besteht der Schaft aus einem proximalen und einem distalen Bereich, wobei im proximalen Schaftbereich der Schaft aus einem steiferen Material hergestellt ist und das Lumen für den Führungsdraht koaxial im Schaft geführt ist, sodass das Lumen zur Ballonspeisung das Lumen für den Führungsdraht ringförmig umgibt und wobei im distalen Schaftbereich der Schaft aus einem flexibleren Material hergestellt ist, und die Lumen zur Ballonspeisung und zur Drahtführung biaxial nebeneinander liegen.

Ein Ballonkatheter dieser Art ist beispielsweise aus der DE GM 91 06 499 bekannt. Der dort gezeigte Ballonkatheter wird zusammen mit einem Führungskatheter und einem Führungsdraht zur perkutanen transluminalen Koronar-Rekanalisation benutzt. Der Katheter wird durch einen Einstich in die Haut in ein Blutgefäss eingeführt und über das Blutgefäss bis zu einer beispielsweise arteriosklerotischen Verengung in den Herzkranzgefässen vorgeschoben. Mit dem auffüllbaren Ballon am Ende des Katheters wird die Verengung dann aufgeweitet.

Der Schaft dieses bekannten Katheters ist aus zwei Längen zusammengesetzt. Die bedienungsseitige, proximale Länge ist aus einem besonders steifen Material hergestellt und ausserdem koaxial, d. h. nach dem System "Schlauch im Schlauch" aufgebaut. Das führt zu einem besonders steifen Schaft in diesem Bereich.

Die bedienungsferne, die distale Schaftlänge ist aus besonders flexiblem Material hergestellt und so aufgebaut, dass die beiden Lumen im Schaft, der Kanal zur Ballonspeisung und der Kanal zur Aufnahme des Führungsdrahtes, in einer gemeinsamen Schafthülle nebeneinander liegen. Diese Bauweise ergibt eine flexiblere Konstruktion, sodass beide Massnahmen zusammen, nachgiebiges Material und flexiblere Bauweise, einen Schaft ergeben, der weich sich allen Blutgefässformen anpasst.

Mit dieser Anordnung soll erreicht werden, dass einerseits das distale Ende des Katheters mit dem zusammengefalteten Ballon sich leicht in die gewundenen Gänge der Herzkranzgefässe einführen lässt, dass andererseits aber gleichzeitig im proximalen, steifen Abschnitt des Schaftes die Schubkräfte zum Vorschieben des Katheters gut übertragen werden.

Wenn der proximale Abschnitt nicht steif genug ist, dann weicht der Schaft beim Vorschieben des Katheters unter den Schubkräften zur Seite hin aus und es entstehen dort, wo der Katheter zur Seite hin ausweicht, besonders hohe Reibungskräfte zwischen Katheter und Seitenwand. Ein Teil der in den Schaft eingebrachten Schubkraft geht dadurch verloren und steht an der Spitze nicht mehr z.B. zum Durchstossen eines Gefässverschlusses zur Verfügung. Dadurch geht auch ein Teil des Tastgefühls für den behandelnden Arzt verloren, indem nämlich ein Teil der Widerstände, die der Arzt beim Vorschieben des Katheters spürt, auf unkontrollierte Reibkräfte zurückzuführen ist und nicht etwa auf Widerstände, die der Arzt bei seiner Behandlung berücksichtigen müsste. Auch legt durch dieses seitliche Ausweichen des Katheterschaftes beim Vorschieben des Katheters die Spitze des Katheters, der Ballon, nicht mehr den gleichen Weg zurück wie das bedienungsseitige Ende. Die Sicherheit und Zuverlässigkeit, mit der der Arzt den Katheter führen kann, erleidet dadurch erhebliche Einbussen.

Es ist wünschenswert, dass ein Katheter dieser Art auch nach dem sogenannten "Monorail"-System eingesetzt werden kann. Bei diesem in der EP 0 203 945 beschriebenen System tritt der von der Spitze an innerhalb des Katheters geführte Führungsdraht nicht erst am bedienungsseitigen Ende des Katheters aus dem Schaft aus, sondern in einem bestimmten, kürzeren Abstand vom Ballon. Das Auswechseln eines Katheters während der Führungsdraht im Blutgefäss mit seinem Ende hinter der Verengung liegen bleibt, ist damit erleichtert. Ohne diese Austrittsöffnung in kurzem Abstand vom Ballon, ohne das "Monorail"-System, muss der Führungsdraht mindestens die gesamte Katheterlänge aus dem Patienten herausragen. Mit dem "Monorail"-System kann der Führungsdraht deutlich kürzer sein und ein Katheterwechsel geht entsprechend einfacher und schneller. Der bedienungsmässige Aufwand, den aus dem Patienten herausstehenden Führungsdraht steril zu halten ist geringer, und der Katheter muss nicht über einen ca. 3 Meter langen Führungsdraht geschoben werden.

Die EP-A-0 441 384 zeigt einen Katheter mit einer "Monorail"-Austrittsöffnung im Schaft des Katheters. Es handelt sich dabei nicht um einen aus zwei Längen verschiedener Steifigkeit zusammengesetzten Schaft. Um bei diesem Schaft eine abgestufte Steifigkeit zu erreichen, muss ein Versteifungsdraht in den Katheter eingeschoben werden. Das führt zu einem zusätzlichen Aufwand für den Versteifungsdraht, seine Montage und für einen Verschlusstopfen am Ende des Lumens in dem der Versteifungsdraht liegt. Ausserdem steht dadurch das entsprechende Lumen nicht mehr für andere Zwecke zur Verfügung, wie z.B. Medikamententransport oder Druckmessung.

Die Erfindung hat sich die Aufgabe gestellt, einen einfach herzustellenden und universell verwendbaren Ballonkatheter der eingangs genannten Art zur Verfügung zu stellen, der nach dem "Monorail"-System einsetzbar ist, dessen Schaftsteifigkeit aber durch die dann notwendige Austrittsöffnung für den Führungsdraht möglichst wenig geschwächt wird.

Diese Aufgabe wird durch den in Anspruch 1 definierten Ballonkatheter gelöst.

Dadurch wird erreicht, dass der Katheter nach dem "Monorail"-System einsetzbar ist. Gleichzeitig wird erreicht, dass die Öffnung für den Führungsdraht die geringst mögliche Schwächung des Katheterschaftes verursacht. Im biaxialen Schaftbereich ist zwar dadurch, dass zur Herstellung einer Öffnung nur eine Wand zu durchstossen ist, eine Durchgangsöffnung für den Führungsdraht leichter herzustellen, dafür stellt aber dort ein grösseres Loch im Schaft eine empfindliche Störung der Schaftstruktur dar. Wegen des dort vorliegenden Schaftaufbaus und dem für diesen Bereich gewählten weichen Material hat der Schaft dort seine geringste Steifigkeit. Durch die Anbringung einer Öffnung in der Schaftstruktur dieses Bereiches würde die Steifigkeit des Schaftes gerade in dem Bereich weiter herabgesetzt, in dem die Steifigkeit ohnehin ihr niedrigstes Niveau hat. Der Schaft knickt dann an dieser Stelle, und es können ernste Probleme auftreten beim Zurückziehen des Katheters aus dem Gefäss. Anstatt im biaxialen Schaftbereich, wo die Öffnung leichter herzustellen wäre, wird die Öffnung aber im koaxialen Bereich durchgeführt. Dort ist das Material des Schaftes steifer und damit widerstandsfähiger gegen Knicken und die Schaftstruktur ist, dadurch dass im Längsschnitt vier Wandungen zur Verfügung stehen stabiler. Ein weiterer Vorteil liegt noch darin, dass bei dieser Anordnung der Durchlass für den Führungsdraht proximal der knickempfindlichen Verbindungsstelle zwischen proximalem und distalem Schaft liegt. Das bedeutet, dass der Führungsdraht noch als zusätzliche Versteifung dieser gefährdeten Stelle zur Verfügung steht. Er überbrückt den kritischen Übergang des flexiblen zum steifen Schaftbereich.Schliesslich ergibt sich noch ein Vorteil daraus, dass bei der erfindungsgemässen Anordnung die Austrittöffnung ohne Bedenken soweit vergrössert werden kann, dass der Führungsdraht beim Einfädeln in den Katheter leicht aus der Öffnung austritt. Im Lumen des Führungsdrahtes brauchen dann keine Hilfen eingebaut werden, die den Austritt des Führungsdrahtes aus dem Lumen erleichtern.

Erfindungsgemäß ist im Bereich der Austrittsöffnung für den Führungsdraht die Wandung des Führungsdrahtlumens mit der Wandung des Ballonlumens abdichtend verbunden, und zur Herstellung der Austrittsöffnung innerhalb des abdichtend verbundenen Segmentes sind sowohl die Wandung des Ballonlumens als auch die Wandung des Führungsdrahtlumens durchbrochen. Dadurch entsteht eine besonders einfach herstellbare Austrittsöffnung, der Führungsdrahtschlauch berührt nur die Wandung des Schaftes anstatt sie zu durchbrechen. Gleichzeitig wird durch die Verdopplung der Wandstärke in dem Berührungsbereich die Festigkeit des Katheterschaftes gerade dort verstärkt, wo wegen der Austrittsöffnung eine Verstärkung angebracht ist. Die Erfindung bedeutet also nicht nur, dass in Zusammenhang mit der Austrittsöffnung eine Schwächung im bereits schwächeren Teil des Katheters vermieden wird, sondern sie bedeutet darüber hinaus, dass eine zu erwartende Schwächung vor ihrem Entstehen durch eine Verstärkung kompensiert wird.

In einer Ausführungsform der Erfindung erstreckt sich das Führungsdrahtlumen ohne Unterbrechung vom äussersten proximalen Ende des Katheters bis zum äussersten distalen Ende des Katheters. Dieses Lumen kann dann vorteilhaft in anderen Anwendungsbereichen noch benutzt werden, z.B. zur Aufnahme eines zusätzlichen, herausnehmbaren u. U. formbaren Versteifungsdrahtes, zum Transport von Flüssigkeiten wie Kontrastmittel, Lösungen zur Reduzierung der Blutgerinnung u. a. und es kann zur Abnahme von Druckmessungen benutzt werden. Wichtig ist, dass mit dieser Massnahme eine Möglichkeit gegeben ist, ohne Lageveränderung des Katheters einen neuen Führungsdraht zu legen, wenn der vorher benutzte versehentlich aus der Austrittsöffnung herausgezogen wurde.

In einer weiteren besonderen Ausführungsform schliesst die im koaxialen Schaftbereich ausgeführte Austrittsöffnung für den Führungsdraht sich an die Verbindungsstelle zwischen proximalem und distalem Schaftbereich an. Diese Massnahme führt dazu, dass das "Monorail"-Führungsdrahtlumen, das Führungdrahtlumen zwischen Austrittsöffnung und distalem Katheterende, relativ kurz bleibt. Die Entwicklung von immer dünneren Ballonkathetern und immer flexibleren distalen Katheterbereichen führt zwar dazu, dass immer ferner liegende Verengungen in den Blutgefässen behandelt werden. Das wiederum führt dazu, dass die distalen flexibleren Katheterbereiche der Katheter immer länger ausgeführt werden. Es liegt aber im Interesse der eingangs erwähnten leichten Handhabung mit Hilfe des "Monorail"-Systems, wenn das "Monorail"-Führungsdrahtlumen relativ kurz bleibt. Die angegebene Massnahme ist insofern ein vorteilhafter Kompromiss in dieser Frage, als die Wurzel des distalen flexiblen Schaftbereiches im Gebrauch vorteilhafterweise innerhalb des in der Aorta liegenden, mit seinem Ende in die Mündung der Herzkranzgefässe reichenden Führungskatheters verbleibt. Die Anordnung der Austrittsöffnung an der Verbindungsstelle, d.h. in dieser Lage des Katheters innerhalb des Führungskatheters, stellt sicher, dass der Führungsdraht immer geführt ist, ausserhalb des Führungskatheters im Führungsdrahtlumen, innerhalb des Katheters jenseits der Austrittsöffnung, im Führungskatheter. Ein Ballonkatheter dieser Art ist auch einfach herzustellen, da die Verbindung der beiden Schlauchwandungen im koaxialen Bereich gleichzeitig mit der Verbindung des koaxialen Schaftbereiches mit dem biaxialen Schaftbereich durchgeführt werden kann.

Wenn die Austrittsöffnung für den Führungsdraht durch einen in axialer Richtung geführten Schnitt durch beide Wandungen, die Wandung des Ballonspeisungslumens und die Wandung des Führungsdrahtlumens erzeugt wird, vereinfacht sich die Fertigung des Ballonkatheters weiter. Durch Schneiden entstehen relativ grosse Späne, verglichen mit Bohren oder Schleifen. Solche grossen Späne sind leichter zu entsorgen, ein Gesichtspunkt der z.B. bei Fertigung in einem Reinraum ins Gewicht fällt. Durch einen Schnitt in axialer Richtung entsteht eine länglich ovale Öffnung im Schaft, die einen schrägen Durchgang des Führungsdrahtes durch die Öffnung zulässt, ohne dass der Draht eine zu scharfe Umlenkung erfährt. Durch den Auslauf des Schnittes in beiden Richtungen werden Spannungsspitzen im Schaft vermieden.

Ein besonders angepasstes Verfahren zur Herstellung eines Ballonkatheters nach der Erfindung besteht darin,
- das Führungsdrahtlumen mit einer Füllung zu versehen, die das Lumen im Bereich der vorgesehenen Austrittsöffnung ausfüllt,
- das Ballonspeisungslumen mit einer Füllung zu versehen, die den Schlauch für den Führungsdraht im Bereich der vorgesehenen Austrittsöffnung an die Wandung des Ballonspeisungslumens presst,
- den Schaft im Bereich der vorgesehenen Austrittsöffnung mit einem Schrumpfelement zu umgeben,
- die Schrumpfung des Schrumpfelementes einzuleiten,
- die durch die Füllungen von innen und das Schrumpfelement von aussen gegeneinander gepressten Wandungen des Führungsdrahtlumens und des Ballonspeisungslumens im Bereich der vorgesehenen Austrittsöffnung thermisch oder durch Bindemittel miteinander zu verbinden,
- die Füllungen jeweils wieder zu entfernen und
- die Austrittsöffnung herzustellen, indem die miteinander verbundenen Wandungen des Führungsdrahtlumens und des Ballonlumens beide durchbrochen werden.
Dieses Verfahren ist besonders einfach und erlaubt zuverlässige und wiederholbare Ergebnisse, ohne dass die ausführende Kraft besondere Vorkenntnisse haben müsste.

Die Erfindung wird im folgenden anhand eines in den Zeichnungen dargestellten Ausführungsbeispiels näher erläutert. Es zeigen:
- Fig. 1: eine Ansicht eines Ballonkatheters nach der Erfindung,
- Fig. 2: eine Ansicht des proximalen Endes eines Ballonkatheters mit Führungskatheter, Führungsdraht und herausnehmbarem Versteifungsdraht,
- Fig. 3: ein Längsschnitt durch den Schaft des Ballonkatheters in Fig. 1 an der Austrittsöffnung für den Führungsdraht,
- Fig. 4: eine Ansicht des Schaftes des Ballonkatheters in Fig. 1 im Bereich der Austrittsöffnung für den Führungsdraht,
- Fig. 5: einen Querschnitt durch den Schaft des Ballonkatheters in Fig. 1 im Bereich des koaxialen Schaftes,
- Fig. 6: einen Querschnitt durch den Schaft des Ballonkatheters in Fig. 1 im Bereich der Austrittsöffnung für den Führungsdraht,
- Fig. 7: einen Querschnitt durch den Schaft des Ballonkatheters in Fig. 1 im Bereich der Verbindungsstelle zwischen koaxialem Schaftbereich und biaxialem Schaftbereich,
- Fig. 8: einen Querschnitt durch den Schaft des Ballonkatheters in Fig. 1 im Bereich des koaxialen Schaftes.

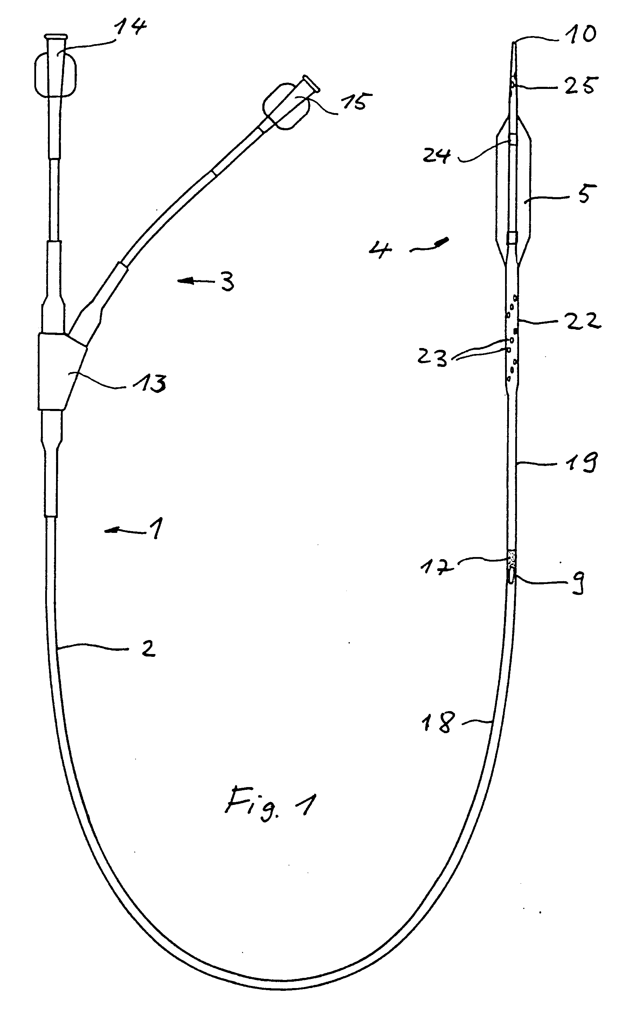

Fig. 1 zeigt einen Ballonkatheter 1 mit einem Schaft 2. Das bedienungseitige, proximale Ende des Katheters ist mit 3 bezeichnet. Das bedienungsferne, distale Ende des Katheters ist mit 4 bezeichnet.

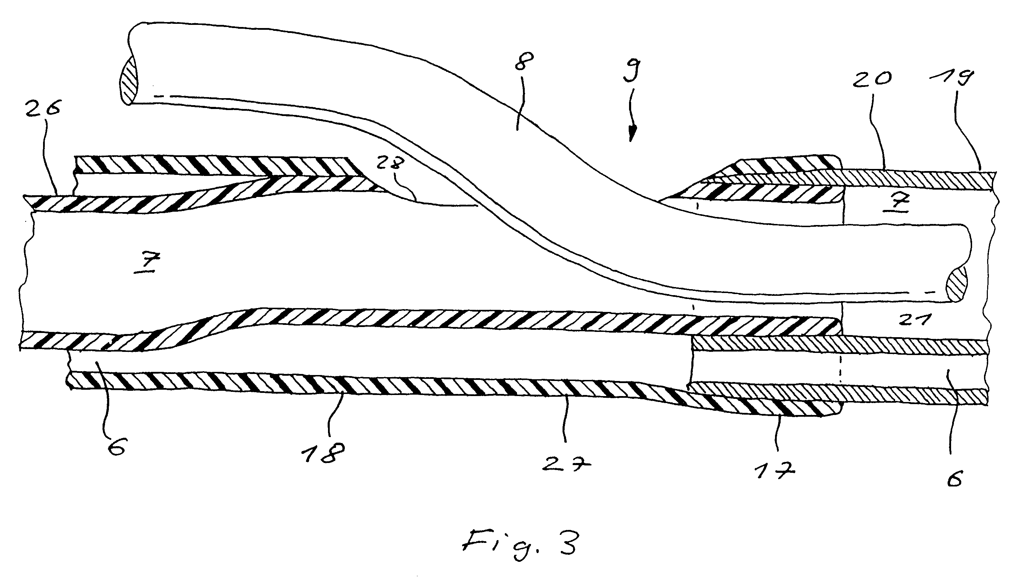

Am distalen Ende 4 des Katheters 1 ist ein Ballon 5 angebracht, der mit einer Flüssigkeit prall aufgebläht werden kann. Der Schaft 2 weist dazu zwei, allgemein Lumen genannte Kanäle auf, ein Lumen 6 zur Ballonspeisung (siehe Fig. 3 bis 6) und ein Lumen 7 für den Führungsdraht 8.

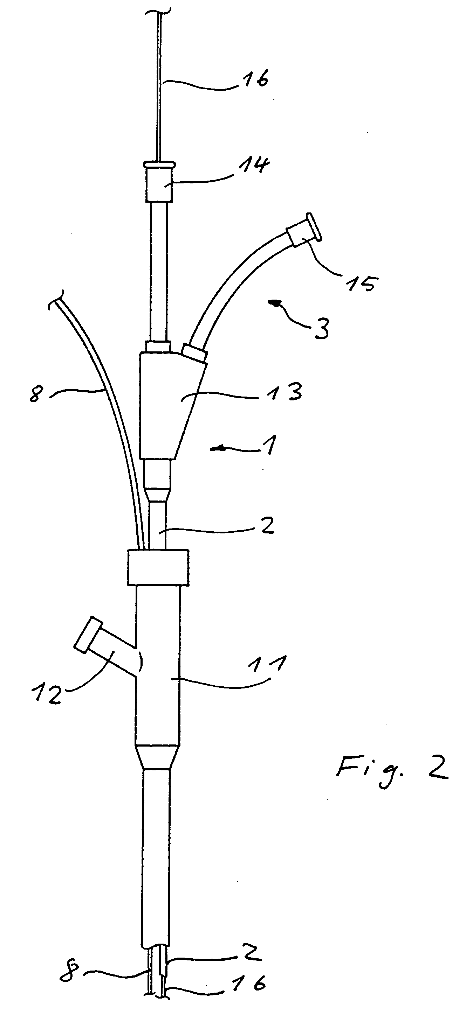

Wie in Fig. 2 dargestellt, wird der Ballonkatheter 1 zusammen mit einem Führungsdraht 8 benutzt. Der Führungsdraht 8 wird zusammen mit dem Katheter 1 oder vor dem Katheter als erstes in das Blutgefäss eingeschoben. Der Führungsdraht 8 ist lenkbar ausgeführt, d.h. er kann eine bestimmte Bogenform an der Spitze aufweisen oder vom Arzt in eine bestimmte Bogenform gebracht werden, in die er, wenn keine Kraft auf ihn einwirkt, immer wieder zurückkehrt. Gleichzeitig ist der Führungdraht torsionssteif. Mit diesen beiden Eigenschaften lässt der Führungsdraht sich in geraden Gefässabschnitten vorschieben, er lässt sich durch die vorgegebene Spitzenform in Abzweigungen hineinlenken, er lässt sich aber auch an Abzweigungen im Gefäss zuverlässig vorbeisteuern, indem nämlich durch Verdrehung des proximalen Drahtendes die vorgegebene Bogenform an der Spitze des Drahtes von der Abzweigung weg zur anderen Gefässeite hin verdreht wird.

Der Führungdraht 8 wird in der in Fig. 2 gezeigten Verwendungsform nach dem in der EP 0 203 945 offenbarten sogenannten "Monorail"-Prinzip benutzt. Das bedeutet, der Führungsdraht 8 liegt vom proximalen Ende 3 des Katheters 1 ausgehend betrachtet, zunächst ausserhalb des Katheters 1 parallel zu ihm. Bei der Öffnung 9 in Fig. 1 tritt der in Fig. 1 nicht eingezeichnete Führungsdraht 8 in den Katheter 1 ein, er wird von da an innen im Katheter 1 geführt und er verlässt den Katheter 1 an der Spitze 10 des Katheters distal vom Ballon 5. In Fig. 2 ist ausser dem Führungsdraht 8 auch der Führungskatheter 11 sichtbar. Er umgibt den Katheter 1 und den Führungsdraht 8 von aussen und dient beiden über eine längere Gefässstrecke als Führung. An seinem proximalen Ende umgibt der Führungskatheter 11 den Katheter 1 und den Führungsdraht 8 mit einer innen liegenden Gummidichtung. Seitlich am Führungskatheter 11 ist ein seitlicher Anschluss 12 angebracht. Er wird für die Zugabe von Kontrastmitteln oder von Medikamenten zu Verhinderung von Blutgerinnung gebraucht oder er dient zur Abnahme von Druckmessungen.

Wie sowohl in Fig. 2 und Fig. 1 sichtbar, ist am proximalen Ende 3 des Katheters 1 ein Abzweig 13 angebracht. In diesem Abzweig 13 wird ein Lumen des Katheters 1 abdichtend weitergeführt in ein Anschlusstück 14. Das andere Lumen mündet in einen abgedichteten Raum im Innern des Abzweiges 13, von dem der zweite Anschluss 15 abgeht. In Fig. 2 ist in das eine Lumen über den Anschluss 14 ein herausnehmbarer Versteifungsdraht 16 eingeführt. Herausnehmbare Versteifungsdrähte werden eingesetzt, um den proximalen Bereich eines Katheters zusätzlich zu versteifen, jedenfalls solange das entsprechende Lumen nicht im vollen Umfang für eine andere Aufgabe gebraucht wird.

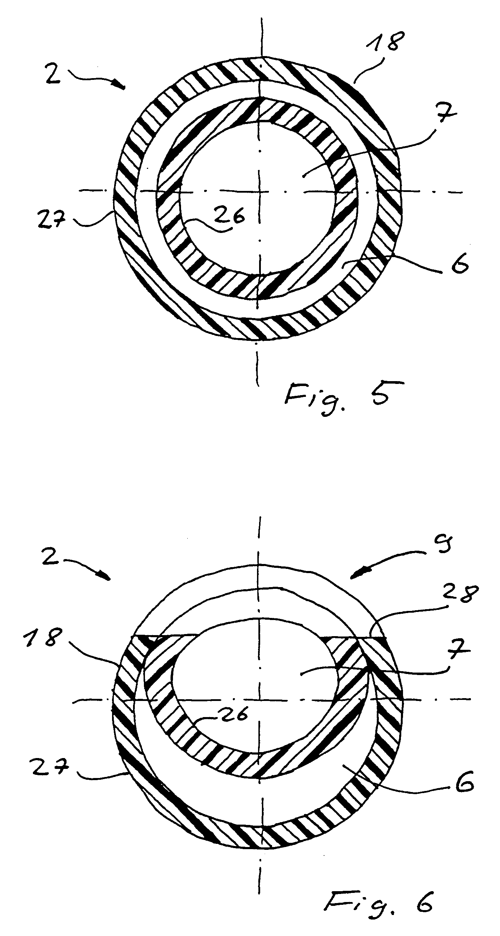

Der Schaft 2 des Katheters 1 in Fig. 1 ist an einer Verbindungsstelle aus einem proximalen Schaftbereich 18 und einem distalen Schaftbereich 19 zusammengesetzt. Im proximalen Bereich 18 ist der Schaft aus einem steiferen Material hergestellt. Ein Querschnitt durch den Schaft im proximalen Schaftbereich ist in Fig. 5 dargestellt. Dort wird deutlich, dass in diesem Bereich des Schaftes 2 das Lumen 7 für den Führungsdraht 8 koaxial, nach dem System "Schlauch in Schlauch" im Schaft geführt ist. Das Lumen 6, durch das der Ballon 5 gespeist wird, umgibt ringförmig das Lumen 7 für den Führungsdraht 8. Diese Konstruktion macht den Schaft 2 noch zusätzlich steif, weil im Längsschnitt damit vier Wandungen zur Verfügung stehen, um zur Biegesteifigkeit beizutragen.

Im distalen Bereich 19 ist der Schaft aus flexiblerem Material hergestellt als im proximalen Bereich 18. Ein Querschnitt durch den Schaft im distalen Schaftbereich 19 ist in Fig. 8 dargestellt. Das Lumen 6 zur Ballonspeisung und das Lumen 7 zur Aufnahme des Führungsdrahtes 8 liegen biaxial nebeneinander mit jeweils eigenen Achsen. Eine gemeinsame Schafthülle 20 umgibt das eine und das andere Lumen, sie sind nur durch eine Trennwand 21 getrennt.

Zwischen der Verbindungsstelle 17 und dem Ballon 5 anschliessend an den Ballon 5 ist der Schaft 2 im distalen Schaftbereich auf einer gewissen Länge 22 weiter kalibriert, sodass das Führungsdrahtlumen 7 dort einen grösseren Durchmesser hat. Auf dieser Länge 22 sind im Schaft 2 mehrere kleine Löcher 23 angebracht, die in das Führungdrahtlumen 7 führen. Die Löcher sind so klein gehalten, dass sie die Festigkeit des Schaftes 2 nicht unterbrechen, gleichzeitig aber noch genügend Blut durchlassen, ohne zu Verstopfungen zu führen.

Innerhalb des Ballons 5 ist das Ballonspeisungslumen 6 aufgeschnitten und ganz entfernt, sodass vom Ballon an nur noch das Führungsdrahtlumen 7 weitergeführt ist. Goldmarkierungen 24 auf dem Schaft innerhalb des Ballons 5 sollen im Röntgenbild deutlich die Position des Ballons 5 anzeigen. Der Ballon 5 ist separat hergestellt und nachträglich mit dem Schaft z.B. durch Verschweissen verbunden. Distal vom Ballon 5 tritt nur noch der Teil des Schaftes 2 aus dem Ballon heraus, der nach der Entfernung des Ballonspeisungslumens 6 übrigbleibt, der Teil des Schaftes, der das Führungsdrahtlumen 7 umgibt.

In diesem distal vom Ballon 5 austretenden Schaftbereich sind wiederum kleine Löcher 25 angebracht. Durch die Löcher 23 und 25 und durch das im Durchmesser grösser kalibrierte Führungsdrahtlumen 7 entsteht über das Führungdrahtlumen 7 eine Verbindung von der proximalen Seite des Ballons 5 zur distalen Seite des Ballons 5. Durch diese Verbindung ist ein gewisser Blutfluss möglich, selbst wenn im Führungsdrahtlumen 7 der Führungsdraht 8 gelegt ist. Der Blutfluss ist entsprechend erhöht, wenn der Führungsdraht 8 aus der Länge 22 zurückgezogen ist. Dieser Blutfluss kann die distal vom Ballon liegenden Gefässe mit Blut versorgen. Das ist ein wichter Gesichtspunkt, weil damit die Behandlung der Verengung in der Ader verlängert werden kann, ohne dass distal vom Ballon gelegene Organe unter zu starker Verminderung der Durchblutung zu leiden haben.

Wenn der Führungsdraht 8 aus der Länge 22 zurückgezogen wird, um den Blutfluss im Lumen 7 zu erleichtern, soll der Führungsdraht nicht versehentlich aus der Öffnung 9 herausgezogen werden. Der Führungsdraht 8 wäre dann für seine Aufgabe verloren. Deshalb ist es günstig, wenn, wie im Ausführungsbeispiel gezeigt, zwischen der Länge 22 und der Öffnung 9 ein deutlicher Abstand eingehalten wird z.B. in der Grössenordnung der halben Länge des distalen Schaftbereiches 19. In der Regel reicht es aber aus, wenn dieser Abstand etwa so lang ist wie die Länge 22 selbst.

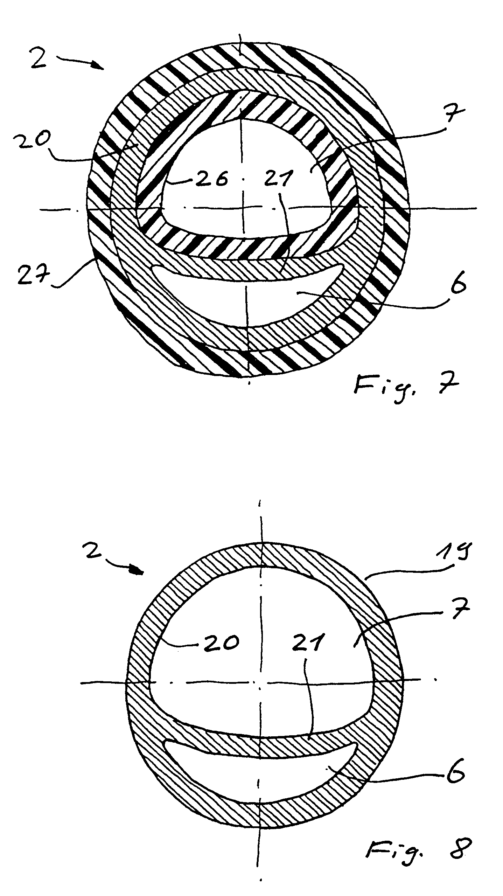

Die Verbindungsstelle 17 zwischen dem proximalen steiferen und dem distalen flexibleren Schaftbereich 18 bzw. 19 ist in Fig. 3 und in Fig. 7 im einzelnen sichtbar. Der innere Schlauch 26 des koaxialen Schaftbereichs 18 ist in das Führungsdrahtlumen 7 des biaxialen distalen Schaftbereiches 19 eingeschoben. Dadurch erstreckt sich das Führungsdrahtlumen 7 ohne Unterbrechung vom äussersten proximalen Ende des Katheters 1 bis zum äussersten distalen Ende des Katheters. Der äussere Schlauch 27 ist über die Schafthülle 20 des biaxialen distalen Schaftbereiches 19 geschoben. Beide Schaftbereiche sind dann miteinander durch Bindemittel oder durch Schweissung verbunden. Ein vorteilhaftes Verfahren besteht darin, die Lumen jeweils mit einer Füllung zu versehen und die Verbindungsstelle 17 mit einem passenden Schrumpfschlauch zu umgeben. Wenn der Schrumpfschlauch entsprechend gewählt ist, reicht die Wärmezufuhr zum Schrumpfen gerade aus, um die beiden Schaftbereiche beim Schrumpfen des Schrumpfschlauches zusammenzupressen und gleichzeitig miteinander zu verschweissen. Der Schrumpfschlauch kann dann wieder entfernt werden.

Die seitliche Öffnung 9 im Schaft 2 für den Führungsdraht 8 ist in den Fig. 3, 4 und 6 in Einzelheiten dargestellt. Aus diesen Zeichnungen geht hervor, dass die Austrittsöffnung 9 für den Führungsdraht 8 nicht im distalen Schaftbereich 19 angeordnet ist, wo der Schaft 2 biaxial ausgeführt ist, sondern vielmehr im proximalen Schaftbereich 18 angeordnet ist, in dem der Schaft 2 koaxial und steifer ausgeführt ist.

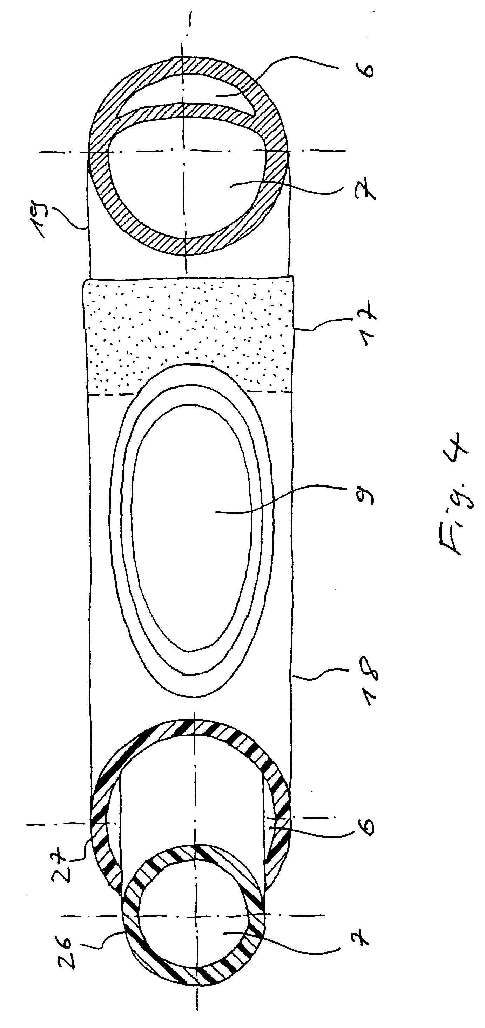

Insbesondere in Fig. 6 ist sichtbar, dass im Bereich der Austrittsöffnung 9 für den Führungsdraht 8 ein Mantelsegment der Wandung des Führungsdrahtlumens 7, d.h. ein Mantelsegment des inneren Schlauches 26 mit der Wandung des Ballonlumens 6, d.h. mit dem äusseren Schlauch 27 abdichtend verbunden ist. Zur Herstellung der Austrittsöffnung 9 innerhalb des abdichtend verbundenen Segmentes ist sowohl die Wandung des Ballonlumens 6, der äussere Schlauch 27, als auch die Wandung des Führungsdrahtlumens 7, der innere Schlauch 26, durch einen Schnitt 28 durchbrochen. Der Schnitt 28 ist dabei in axialer Richtung geführt und durchdringt beide Wandungen, die Wandung des Ballonspeisungslumens und die Wandung des Führungsdrahtlumens gleichzeitig. Er ergibt damit ein länglich ovales Loch wie in der Ansicht in Fig. 4. Die so erzeugte Ausschnittsform führt zu keinen Spannungspitzen im Katheterschaft. Diese Ausschnittsform kann so gross und so lang gestaltet werden, dass der Führungsdraht 8 beim Einfädeln in den Katheter leicht aus dem Schaft 1 austritt, ohne dass im Lumen 7 selbst Austrittshilfen, wie z.B. eine Rampe oder ähnliches, angeordnet werden müssen. Die Austrittsöffnung 9 für den Führungsdraht 8 im koaxialen Schaftbereich 18 schliesst sich so nah an die Verbindungsstelle 17 zwischen proximalem und distalem Schaftbereich 18, 19 an, dass die Verbindungsstelle 17 und die abdichtende Verbindung für die Austrittsöffnung 9 in einem Arbeitsgang hergestellt werden können.

Ein besonders geeignetes Verfahren zur Herstellung einer Austrittsöffnung für den Führungsdraht in einem Ballonkatheter nach der Erfindung besteht darin,
- das Führungsdrahtlumen 7 mit einer Füllung wie z.B. einem Schlauch aus geeignetem Kunststoff zu versehen, die das Lumen 7 im Bereich der vorgesehenen Austrittsöffnung 9 ausfüllt,
- das Ballonspeisungslumen 6 mit einer Füllung, zum Beispiel einem Profil aus geeignetem Kunststoff, zu versehen, die den Schlauch 26 für den Führungsdraht 8 im Bereich der vorgesehenen Austrittsöffnung 9 an die Wandung des Ballonspeisungslumens 6, von innen an den Schlauch 27 presst,
- den Schaft 2 im Bereich der vorgesehenen Austrittsöffnung 9 mit einem Schrumpfschlauch zu umgeben,
- die Schrumpfung des Schrumpfschlauches durch Erwärmung einzuleiten,
- die durch die Füllungen von innen und das Schrumpfelement von aussen gegeneinander gepressten Wandungen des Führungsdrahtlumens 7 und des Ballonspeisungslumens 6 im Bereich der vorgesehenen Austrittsöffnung 9 thermisch z.B. durch Schweissen oder durch Bindemittel miteinander zu verbinden,
- die Füllungen jeweils wieder zu entfernen,
- den Schrumpfschlauch zu entfernen und
- die Austrittsöffnung 9 herzustellen, indem die miteinander verbundenen Wandungen des Führungsdrahtlumens 7, d.h. der Schlauch 26, und des Ballonlumens 6, d.h. der Schlauch 27, beide mit einem Schnitt 28 durchbrochen werden.

## Patentansprüche

1. Ballonkatheter mit einem Schaft (2), an dessen distalem Ende (4) ein Ballon (5) angebracht ist, wobei der Schaft (2) ein Lumen (7) aufweist, durch das ein Führungsdraht (8) geführt wird, mit dem der Katheter (1) geführt und gesteuert wird, dabei besteht der Schaft (2) aus einem proximalen (18) und einem distalen Bereich (19), wobei im proximalen Schaftbereich (18) der Schaft (2) aus einem steiferen Material hergestellt ist und das Lumen (7) für den Führungsdraht (8) koaxial im Schaft (2) geführt ist, sodass ein Lumen (6) zur Ballonspeisung das Lumen (7) für den Führungsdraht (8) ringförmig umgibt und wobei im distalen Schaftbereich (19) der Schaft (2) aus einem flexibleren Material hergestellt ist, und die Lumen (6,7) zur Ballonspeisung und zur Drahtführung biaxial nebeneinander liegen, dadurch gekennzeichnet, dass im proximalen Schaftbereich (18) des Schaftes (2) des Katheters (1), wo der Schaft (2) steifer und koaxial ausgeführt ist, die Wandung (26) des Führungsdrahtlumens (7) mit der Wandung (27) des Ballonlumens (6) abdichtend verbunden ist und dass im proximalen, und abdichtend verbundenen Bereich seitlich eine Austrittsöffnung (9) für den Führungsdraht (8) vorgesehen ist.

2. Ballonkatheter nach Anspruch 1, dadurch gekennzeichnet, dass das Führungsdrahtlumen (7) sich ohne Unterbrechung vom äussersten proximalen Ende des Katheters (1) bis zum äussersten distalen Ende des Katheters erstreckt.

3. Ballonkatheter nach einem der Ansprüche 1 bis 2, dadurch gekennzeichnet, dass die im koaxialen Schaftbereich (18) ausgeführte Austrittsöffnung (9) für den Führungsdraht (8) sich an die Verbindungsstelle (17) zwischen proximalem (18) und distalem (19) Schaftbereich anschliesst.

4. Ballonkatheter nach einem der Ansprüche 1 bis 3, dadurch gekennzeichnet, dass die Austrittsöffnung (9) für den Führungsdraht (8) durch einen in axialer Richtung geführten Schnitt (28) durch beide Wandungen, die Wandung (27) des Ballonspeisungslumens (6) und die Wandung (26) des Führungsdrahtlumens (7), erzeugt wird.

5. Verfahren zur Herstellung einer Austrittsöffnung (9) für den Führungsdraht (8) in einem Ballonkatheter nach einem der Ansprüche 1 bis 4, bei dem
- das Führungsdrahtlumen (7) mit einer Füllung versehen wird, die das Lumen im Bereich der vorgesehenen Austrittsöffnung (9) ausfüllt,
- das Ballonspeisungslumen (6) mit einer Füllung versehen wird, die den Schlauch (26) für den Führungsdraht (8) im Bereich der vorgesehenen Austrittsöffnung (9) an die Wandung (27) des Ballonspeisungslumens (6) presst,
- der Schaft (2) im Bereich der vorgesehenen Austrittsöffnung (9) mit einem Schrumpfelement umgeben wird,
- die Schrumpfung des Schrumpfelementes eingeleitet wird,
- die durch die Füllungen von innen und das Schrumpfelement von aussen gegeneinander gepressten Wandungen (26) des Führungsdrahtlumens (7) und des Ballonspeisungslumens (6) im Bereich der vorgesehenen Austrittsöffnung (9) thermisch oder durch Bindemittel miteinander verbunden werden,
- die Füllungen jeweils wieder entfernt werden und
- die Austrittsöffnung (9) hergestellt wird, indem die miteinander verbundenen Wandungen (26) des Führungsdrahtlumens (7) und des Ballonlumens (6) beide durchbrochen werden.

## Claims

1. Balloon catheter with a shank (2) on whose distal end (4) a balloon (5) is arranged, the shank (2) having a lumen (7) through which there is guided a guide wire (8) with which the catheter (1) is guided and controlled, the shank (2) consisting of a proximal region (18) and a distal region (19), wherein the shank (2) is produced from a stiffer material in the proximal shank region (18) and the lumen (7) for the guide wire (8) is guided coaxially in the shank (2) so that a lumen (6) for supplying the balloon annularly surrounds the lumen (7) for the guide wire (8) and wherein the shank (2) is produced from a more flexible material in the distal shank region (19) and the lumens (6, 7) for the supply of the balloon and for wire guidance lie biaxially next to one another, characterised in that in the proximal shank region (18) of the shank (2) of the catheter (1) where the shank (2) is stiffer and is coaxial in design, the wall (26) of the guide wire lumen (7) is connected in a sealing manner to the wall (27) of the balloon lumen (6) and in that an outlet orifice (9) for the guide wire (8) is provided laterally in the proximal region which is connected in a sealing manner.

2. Balloon catheter according to Claim 1, characterised in that the guide wire lumen (7) extends without interruption from the outermost proximal end of the catheter (1) to the outermost distal end of the catheter.

3. Balloon catheter according to one of Claims 1 to 2, characterised in that the outlet orifice (9) formed in the coaxial shank region (18) for the guide wire (8) adjoins the connecting point (17) between proximal shank region (18) and distal shank region (19).

4. Balloon catheter according to one of Claims 1 to 3, characterised in that the outlet orifice (9) for the guide wire (8) is produced by a cut (28) guided axially through the two walls, the wall (27) of the balloon supply lumen (6) and the wall (26) of the guide wire lumen (7).

5. Process for the production of an outlet orifice (9) for the guide wire (8) in a balloon catheter according to one of Claims 1 to 4 in which the guide wire lumen (7) is provided with a filling which fills the lumen in the region of the proposed outlet orifice (9), the balloon supply lumen (6) is provided with a filling which presses the tube (26) for the guide wire (8) in the region of the proposed outlet orifice (9) against the wall (27) of the balloon supply lumen (6), the shank (2) is surrounded by a shrink element in the region of the proposed outlet orifice (9), the shrinkage of the shrink element is initiated, the walls (26) of the guide wire lumen (7) and of the balloon supply lumen (6) pressed against one another from the interior by the fillings and from the exterior by the shrink element are connected to one another thermally or by a binder in the region of the proposed outlet orifice (9), the fillings are each removed again and the outlet orifice (9) is produced in that the walls (26) of the guide wire lumen (7) and of the balloon lumen (6) connected to one another are both perforated.

## Revendications

1. Cathéter à ballonet, comprenant une tige (2) à l'extrémité distale (4) de laquelle est prévu un ballonet (5), la tige (2) présentant un lumen (7) à travers lequel passe un fil de guidage (8) qui guide et dirige le cathéter (1), la tige (2) étant constituée d'une région proximale (18) et d'une région distale (19), la tige (2) étant réalisée dans une matière plus rigide dans la région proximale (18) et le lumen (7) pour le fil de guidage (8) étant guidé coaxialement dans la tige (2), un lumen (6) d'alimentation du ballonet (5) entourant à la manière d'un anneau le lumen (7) pour le fil de guidage (8), la région distale (19) de la tige (2) étant réalisée en une matière plus flexible et les lumens (6, 7) pour l'alimentation du ballonet et le guidage du fil étant juxtaposés biaxialement,
caractérisé en ce que, dans la région proximale (18) de la tige (2) du cathéter (1), où la tige (2) est plus rigide et coaxiale, la paroi (26) du lumen (7) pour le fil de guidage (8) est reliée de manière étanche à la paroi (27) du lumen (6) d'alimentation du ballonet et que, dans la région proximale, reliée de manière étanche, il est prévue latéralement une ouverture de sortie (9) du fil de guidage (8).

2. Cathéter à ballonet selon la revendication 1,
caractérisé en ce que le lumen (7) pour le fil de guidage s'étend sans interruption de l'extrémité proximale extrême du cathéter (1) jusqu'à l'extrémité distale extrême du cathéter.

3. Cathéter à ballonet selon l'une des revendications 1 à 2,
caractérisé en ce que l'ouverture de sortie (9) pour le fil de guidage (8) pratiquée dans la région coaxiale (18) de la tige fait suite au point de connexion (17) entre les régions proximale (18) et distale (19) de la tige.

4. Cathéter à ballonet selon l'une des revendications 1 à 3,
caractérisé en ce que l'ouverture de sortie (9) pour le fil de guidage (8) est pratiquée par une incision axiale (28) à travers les deux parois, à savoir la paroi (27) du lumen (6) d'alimentation du ballonet et la paroi (26) du lumen (7) pour le fil de guidage.

5. Procédé pour réaliser une ouverture de sortie pour le fil de guidage (8) d'un cathéter à ballonet selon l'une des revendications 1 à 4, dans lequel
- on introduit dans le lumen (7) pour le fil de guidage un moyen de remplissage qui remplit le lumen dans la région de l'ouverture de sortie (9) prévue,
- on introduit dans le lumen (6) d'alimentation du ballonet un moyen de remplissage qui pousse le tuyau (26) pour le fil de guidage (8) dans la région de l'ouverture de sortie (9) prévue, contre la paroi (27) du lumen d'alimentation du ballonet,
- la tige (2) est entourée, dans la région de l'ouverture de sortie (9) prévue, d'un élément contractible,
- la contraction dudit élément est initiée,
- les parois (26) du lumen (7) pour le fil de guidage et du lumen (6) d'alimentation du ballonet, poussées l'une contre l'autre depuis l'intérieur par les moyens de remplissage et depuis l'extérieur par l'élément contractible, sont reliées entre elles thermiquement ou par un liant dans la région de l'ouverture de sortie (9) prévue,
- les moyens de remplissage respectifs sont enlevés, et
- l'ouverture de sortie (9) est pratiquée en perçant les parois reliées entre elles du lumen (7) pour le fil de guidage et du canal (6) d'alimentation du ballonet.
